# EUROPEAN PATENT APPLICATION

(11) **EP 2 107 124 A1**
(43) Date of publication of application: **07.10.2009**
(21) Application number: 07019659.7
(22) Date of filing: 08.10.2007
(51) Int. Cl.: C12Q 1/68

(54) **Nuclease on chip**

(71) Applicant: EPPENDORF AG, 22339 Hamburg (DE)
(72) Inventor: Bachmann, Til, Edinburgh EH9 1JJ (GB); Barl, Timo, Edinburgh EH16 4SB (GB); Köhn, Heinz Gerhard, 22339 Hamburg (DE)
(74) Representative: Becker Kurig Straus

(57) **Abstract**

The present invention pertains to a method for determining point mutations on micro-arrays/biochips using a nuclease capable to selectively digest hybridized DNA strands into two DNA fragments, said hybridized DNA strands containing a nucleotide mismatch or nucleotide deletion. The occurrence and/or location of such DNA fragments are indicative for a point mutation.

## Description

The present invention pertains to a method for determining point mutations on micro-arrays/biochips using a nuclease capable to selectively digest hybridized DNA strands into two DNA fragments, said hybridized DNA strands containing a nucleotide mismatch or nucleotide deletion. The occurrence and/or location of such DNA fragments are indicative for a point mutation.

In recent years, particularly the Human Genome project, the ongoing research in revealing bacterial resistance and bacterial genome projects associated therewith require sequencing not only of large numbers of nucleotide sequences but also the efficient and reliable determination of particular point mutations.

Sequencing of nucleotide sequences as well as determination of point mutations may be carried out using micro-arrays or biochips in which nucleotides of defined lengths and/or known sequences have been applied at predefined sites of a solid support. The solid support is than contacted to a second nucleotide linked to an identifiable marker under conditions enabling binding of the second nucleotide preferentially to a nucleotide complementary thereto. After removal of non-hybridized nucleotides, detection of the marker at a defined position of the biochip or micro-array is an indication of the presence or absence of a property to be investigated.

As regards the current sequence data, it was found that certain characteristics of higher organisms, such as the tendency towards development of certain diseases or, in the case of the bacteria, the development of resistance to antibiotics, are based on a change only in a small number and in some cases only a single nucleotide point mutation ("single nucleotide polymorphism" SNP) in the sequence of the respective gene responsible therefore or involved therein. Therefore, there is an increased need for determining or demonstrating such changes in the genome of organisms in order to conduct, for example, population studies or forensic medical examinations or in order to demonstrate the presence of antibiotic-resistant bacteria.

Such minor changes of a nucleotide sequence may be detected by means of various methods such as, for example, sequencing the nucleotide sequences of interest, restriction fragment length polymorphism analyses or allele-specific hybridization using longer probe sequences. Due to an inherent methodological and time expenditure in performance such methods are, however, unsuitable for screening of larger numbers of samples.

The characterization of a target sequence in a higher throughput may be for example obtained by comparison of a given pattern with a reference sequence. This technique was applied to the identification of a Mycobacterium tuberculosis gene (WO97/29212 and WO98/28444), wherein one target sequence is cut into pieces before its hybridization on an oligonucleotide array comprising sets of capture nucleotide sequences of less than 30 nucleotides, each set comprising 4 oligonucleotides with one interrogation position for each of the 4 bases (A, T, G, C). This means that for the identification of a target sequence of 20 bases, the array comprises 80 different oligonucleotides. The method is also suited for analysis of two different sequences that may differ by a single nucleotide (identification of SNPs or genotyping).

WO 2006/138257 discloses random arrays of single molecules, provided for carrying out large scale analyses of biomolecules and SNP detection. The arrays may comprise concatemers of DNA fragments that are randomly disposed on a regular array of discrete spaced apart regions, such that substantially all such regions contain no more than a single concatemer.

EP 1 726 648 pertains to A DNA array for detecting a single nucleotide polymorphism of a gene on a solid support. The array comprises a first probe spots group consisting of one or more probe spots each containing one or more probes hybridisable with a polynucleotide of the gene, in which the probes are exactly complementary to the first polymorphism pattern of the gene, and a second probe spots group consisting of one or more probe spots each containing one or more probes hybridisable with the polynucleotide of the gene, in which the probes are exactly complementary to the second polymorphism pattern of the gene, wherein probe lengths in the probe spots is different from each other.

A method for the multiparallel or individual detection of nucleotide polymorphisms on a polydimensional array is disclosed in US 2006/127932 in which hybridization of a probe molecule with a sample molecule ensues in a hybridization field on the array that is separated from surrounding hybridization fields.

US 2006/024715 relates to method and an array for genotyping large numbers of human SNPs in parallel based on a set of human SNPs, known to be biallelic in at least two populations. Allele specific perfect match probes and genotyping probe sets are provided for each allele of each biallelic SNP in a set of human SNPs that is useful for genetic analysis within and across populations. Probe sets that include perfect match and mismatch probes are provided. The probe sets are suitable for inclusion in an array.

A disadvantage of the above mentioned methods for determining the presence of point mutations resides in labelling, wherein the target molecule is labelled and the probe molecule remains unlabelled. So far, labeling was performed by incorporation of expensive dye-labelled nucleotides during PCR. This gold standard step in DNA micro-array experiments is cost-intensive and results in a decreased yield of polymerase chain reaction (PCR) products. PCR is always a critical step, particularly in case quantitative analysis has to be performed.

An object of the present invention is, therefore, to overcome the drawbacks of the current technology and provide a method allowing the determination of point mutations on a micro-array in an inexpensive manner, optionally without performing amplification and/or labeling step.

This object is achieved by provision of a method for determination of a point mutation on a micro-array, in which a nucleotide sequence obtained from a biological sample is contacted with a carrier under conditions permitting hybridization of complementary strands. On the surface of said carrier at least one capture probe is attached by means of a spacer molecule of a defined length. The carrier is further contacted with a nuclease under conditions permitting the nuclease to digest hybridized strands in two or more DNA fragments. Occurrence and/or location of said at least two DNA fragments are indicative of the presence of a point mutation.

During the extensive studies leading to the present invention, it was unexpectedly found that particular endonucleases are capable of digesting hybridized nucleotide strands not only in solution, in which the strands are free accessible to the enzyme, but also in case of attachment of said strands on a surface of a solid support, i.e. under conditions of limited accessibility. In addition, it has been found that endonucleases' activity towards single nucleotide strands may be essentially or completely suppressed and /or eliminated in case said single nucleotide strand is attached to a support and is of a defined length. Hence it is even possible to attach the at least one capture probe to the surface via a single-stranded nucleotide spacer molecule of defined length, which stays single-stranded even after hybridization. On the basis of this finding, a novel and rapid DNA micro-array determination system was developed, by which SNPs or a small number of changes in a nucleotide sequence of interest may be reliably determined.

In the present description the following definitions apply:

The terms "micro-array" and "biochip" may be used interchangeably in the present invention and refer to a multiplicity of different nucleotide sequences attached (preferably through a single terminal covalent bond) to a carrier.. Both terms may refer to the entire collection of oligonucleotides on the carrier or to a subset thereof. The carrier is generally composed of a solid surface which may be selected from the group consisting of glasses, electronic devices, silicon supports, silica, metal, polymers or mixtures thereof prepared in format selected from the group of slides, discs, gel layers, laminates and/or beads. The carrier may be rigid or flexible.

The term "mutation" refers to changes in the base pair sequence of genetic material (either DNA or RNA).
A "point mutation" or "substitution" is a type of mutation that causes the replacement of a single base nucleotide with another nucleotide. Both insertions and deletions of a single base pair fall under the term "point mutation". A prominent example for a point mutation is the single nucleotide polymorphism (SNP) in which a DNA sequence variation occurs when a single natural nucleotide - A, T, C, or G - in an oligonucleotide or another nucleotide sequence differs between members of a species.

The term "biological sample" describes any soluble and non-soluble substance extracted, excreted, or secreted from an organism or tissue of an organism, in particular nucleic acid containing substances. Samples of particular relevance to the present invention include but are not limited to whole blood, serum, plasma, urine, synovial fluid, cerebrospinal fluid, saliva, mucosal swabs, tissue extracts, organ extracts and cell extracts. The biological sample may be any medical/veterinary sample, in particular a medical/veterinary sample suspected to carry a point mutation associated with resistance phenomena against drugs (eg. antimicrobial or anti-tumour), any forensic sample, any sample taken from an organism (animals and/or plants) suspected to be a gene modified organism (GMO), and /or any kind of microbial entities (microorganisms) as such, e.g. bacteria ,fungi, viruses, single-cell parasites or parasitic stages, or cells infected with anyone of those and comprising parts thereof. The biological sample may be for example any material supposed to contain a pathogenic strain - a "strain" is a genetic variant or subtype of a microorganism- suspected to exhibit a resistance towards an antimicrobial agent, for example antibiotics, antiviral, anti-fungal and antiparasitic drugs, which resistance may be detected via SNPs. For some applications, it may be appropriate to transfer the biological sample into a medium suitable for the growth of said strain, e.g. on LB agar plates in case of a pathogenic *E. coli* strain. Examples of a pathogenic strains of interest for the present invention are those causing infections, including nosocomial infections. Of particular interest are for example *Streptococcus aureus, Pseudomonas aeruginosa, Escherichia coli (E. coli) (in particular 0157:H7), Neisseria meningitidis, Streptococcus pneumoniae, staphylococcus epidermis, Mycobacterium tuberculosis, Mycobacterium africanum, M microti and M. bovis.*

Pathogenic strains are often the result of pathoadaptive mutations. The term "pathoadaptive mutation" refers to mutation which improve the fitness of pathogenic species by modification of traits that interfere with factors required for survival in host tissues. Pathoadaptive mutations lead to an enhanced pathogenicity (Sokurenko et al. (1999), Pathoadaptive mutations: gene loss and variation in bacterial pathogens. Trends Microbiol. 7(5):191-5). In *E.coli* a number of pathoadaptive mutations are known. *E. coli* is responsible for three types of infections in humans: urinary tract infections (UTI), neonatal meningitis, and intestinal diseases (gastroenteritis). Uropathogenic *E. coli.* causing UTI colonize the bladder with the aid of specific adhesins. The adhesin that has been most closely associated with uropathogenic *E. coli* is the P fimbria (or pyelonephritis-associated pili [PAP] pili). The letter designation is derived from the ability of P fimbriae to bind specifically to the P blood group antigen which contains a D-galactose-D-galactose residue. The fimbriae bind not only to red cells but to a specific galactose dissaccharide that is found on the surfaces uroepithelial cells in approximately 99% of the population. Uropathogenic strains of *E. coli* possess other determinants of virulence in addition to P fimbriae. *E. coli* with P fimbriae also possess the gene for Type 1 fimbriae, and there is evidence that P fimbriae are derived from Type 1 fimbriae by insertion of a new fimbrial tip protein to replace the mannose-binding domain of Type 1 fimbria. In any case, Type 1 fimbriae provide a supplementary mechanism of adherence or play a role in aggregating the bacteria to a specific manosyl-glycoprotein that occurs in urine. Pathoadaptive polymorphisms in FimH, which alters the binding behaviour of the binding-subunit of Type 1 fimbriae are known. The fim H gene is thus a target for the present invention
A further target for the present invention is the *gyrA* gene coding for the Topoisomerease II *(Gyr A).* A polymorphisms in the *Gyr A* gene which corresponds to amino acid positions 83 and 87 is the main cause of fluoroquinolone resistance.

As used in present invention, the term "probe" or "capture probe" is defined as an oligonucleotide capable of binding to a target nucleic acid of complementary sequence through one or more types of chemical bonds, usually through complementary base pairing, usually through hydrogen bond formation. As used herein, an oligonucleotide probe may include natural (i.e. A, G, C, or T) or any modified base (7-deazaguanosine, inosine, etc.) known in the state of the art. In addition, the bases in oligonucleotide probe may be joined by a linkage other than a phosphodiester bond, such as e.g. peptide bonds, so long as it does not interfere with hybridization. Hence a probe may also be a peptide nucleic acid (PNA) or a locked nucleic acid (LNA) or a LNA-modified probe. In one embodiment of the present invention, the probe is labeled. In another embodiment of the invention the probe is not labeled, and instead the target carries a label.

The term "target nucleic acid" or in short "target" refers to a nucleic acid (DNA and/or RNA), to which the oligonucleotide probe specifically hybridizes. The target are nucleotides of any length, i.e. up to the length of the entire genome. Advantageously, the length of the target is selected as being of limited length preferably between 15 and 100 bases, preferably between 100 and 200 bases, preferably between 100 and 400 bases and more preferably between 100 and 800 bases. If the target is an RNA, a reverse transcription step is included followed by an amplification reaction. If the target is a DNA , the target may also be amplified prior to the hybridisation reaction. Amplification reactions are well known in the art; preferably the amplification reaction is selected from the group of PCR, LCR, CPT, NASBA, ICR and Avalanche DNA techniques. In one embodiment of the invention, the target is labeled. The labeling can be performed with techniques known to the skilled artisan (WO9727317). For example, the label may be introduced during the amplification recaction, alternatively a labeled moiety may be ligated with a ligase to the target, or a kinase or a transferase or a similar enzyme may be used to introduce a label.

The term "perfectly matching" or "exactly matching" refers to a probe that has a sequence that is perfectly complementary to a particular target sequence, whereas the term "matching" designates hybridization between probe and target molecule in which there is at least one mismatch present. Preferably only one mismatch is present in the matching hybridization product. The mismatch position may be placed throughout the probe, i.e. close to the 3', and 5' ends as well in the center of the probe. Preferably the mismatch position lies in the center of the probe. If the mismatch position is close to one of the ends of the probe, the distance to the closest end -i.e. the number of bases between the 3'end and the mismatch and the number of bases between 5' end and mismatch, respectively- has to be chosen based on the geometry of the active center of the enzyme. Depending on the endonuclease used, the preferred minimum distance varies.

In the context of this application, the term "nucleotide" includes DNA conventionally having adenine, cytosine, guanine and thymine as bases and deoxyribose as the structural sugar element. Furthermore, a nucleotide can, however, also comprise any modified base known to the skilled artisan, which is capable of base pairing using at least one of the aforesaid bases. Further included in the term "nucleotide" are the derivatives of the aforesaid compounds, in particular derivatives being modified with dyes or radioactive markers.

The term "nucleotide sequence" as used herein refers to nucleotides present in nucleic acids compared with the bases of said nucleic acid, and includes nucleotides comprising usual or modified bases as above described. References to nucleotide(s), oligonucleotide(s), polynucleotide(s) and the like include analogous species wherein the sugar-phosphate backbone is modified and/or replaced, provided that its hybridization properties are not destroyed.

The phrase "hybridizing to" refers to the binding, duplexing or hybridizing of a molecule substantially to or only to a particular nucleotide sequence or sequences under stringent conditions when that sequence is present in a complex mixture of DNA or fragments thereof. Also other hybrid systems like PNA-DNA and LNA-DNA are encompassed. Suitable hybridisation protocols may be derived e.g. from Maniatis, A Laboratory Handbook, Cold Spring Harbor, 1992 and optionally modified according to knowledge of the skilled person.

The expression "stringency conditions" refers to the different conditions that are used to facilitate hybridization of a probe nucleic acid sequence to a target nucleic acid sequence.

In general, "low stringency " conditions are conditions that favor hybridization between probe and target nucleic acid sequence containing mismatched bases relative to the probe.

Low stringency conditions generally refer to hybridization effected at lower temperature (42.degree. C., e.g., instead of 65.degree. C.) and lower salt concentrations.

By contrast, "high stringency" conditions only facilitate hybridization before a probe and a target nucleic acid sequence containing no mismatched bases, or very few relative to the probe nucleic acid sequence. High stringency conditions typically refer to hybridizations effected at higher temperatures, e.g., about 65.degree. C., or higher salt concentrations. The meaning of "high stringency " and "low stringency " is well known to those skilled in the art. (See, Molecular Cloning: A Laboratory Manual, 2.sup.nd Ed. Sambrook et al, 1987.)

The term "endonuclease" relates to enzymes cleaving the phosphodiester bond within a polynucleotide chain and exhibiting the capability to specifically digest complementary nucleotide strands exhibiting a single mismatch, as main reactivity. Thus the endonuclease is preferentially a mismatch-specific endonuclease cutting heteroduplexes. Of interest are mismatch-specific endonucleases which have a mismatch bias. In a preferred mismatch bias the heteroduplex contains at the mismatch position a cytosine. Of particular interest are mismatch-specific endonucleases derived from thermophile organisms. Such endonucleases are stable at higher temperatures. In particular they remain active within a temperature range of 40°C to about 95 °C. If an endonuclease has as undesired secondary activity the capability to cleave/digest single stranded DNA. said activity may essentially be suppressed/eliminated by attaching the capture probe to a carrier by means of a spacer molecule of defined length. Upon hybridization only the oligonucleotide spacer remains single-stranded. Due to is defined length it is not a substrate for the single-strand activity of the endonuclease. The term "essentially suppressed/eliminated" defines hereby a preference of nuclease to cleave double stranded DNA in that in a mixture of double stranded and single stranded DNA the signal obtained by cleavage of double stranded DNA prevails, making at least 70% of the overall signal, preferably at least 80% of the signal, more preferably 90% of the signal and most preferably 95%, 96%, 97%, 98% or 99% of the signal. The mismatch-specific endonuclease is preferentially selected from the group of phage T7 endonuclease I (Babon et al., (2003) The use of resolvases T4 endonuclease VII and T7 endonuclease I in mutation detection; Mol. Biotechnol. 23, 73-81) (available from New England Biolabs^{®)}, T4 endonuclease VII (Mikhailov and Rohrmann (2002), Binding of the baculovirus very late expression factor 1 (VLF-1) to different DNA structures, BMC Mol. Biol. 3, 14 ) (available from USB Corp.), modified bacterial endonuclease V (US2007099216 A1; incorporated herewith by reference) (Huang et al. (2002), An endonuclease/ligase based mutation scanning method especially suited for analysis of neoplastic tissue; Oncogene 21, 1909-1921) and nucleases from the Cel family of nucleases, in particular plant Cell and Surveyor nucleases (available from Transgenomic Inc.)(Qiu et al. (2004), Mutation detection using Surveyor nuclease; Biotechniques 36, 702-707; Kulinski et a.l (2000), CEL I enzymatic mutaion detection assay; Biotechniques 29, 44-46, 48 ). Of particular interest are also those mismatch-specific endonucleases isolated from thermophile organisms, like the modified bacterial endonuclease V derived from *Thermotoga maritima* (US2007099216 A1; incorporated by reference). Of particular interest are also the mismatch specific Surveyor nucleases (available from Transgenomic).

The term "DNA fragment(s)" refer(s) to the reaction product(s) of cleavage/digestion of hybridized DNA strands with an endonuclease. At least one fragment is being released from the carrier. The fragments might dissociate into single-stranded species. Depending on the original position of the label, the label stays attached to the fragment being released from the carrier and thus leaves the carrier together with the released fragment. Alternatively the label remains on the carrier attached to the fragment remaining on the carrier. If the label is a dye quencher pair, the pair is separated through the endonuclease cleavage.

The terms "background" or "background signal intensity" refers to hybridization signals resulting from non-specific binding, or other interactions, between the labeled target nucleic acids and components of the nucleotide array (e.g., the nucleotide probes, control probes, the array substrate, etc.).

Detectable labels suitable for use in the present invention include any composition detectable by spectroscopic, colorimetric, photochemical, biochemical, immunochemical, electrical, electroluminescent, chemiluminscent, magnetic, optical or chemical means. Useful labels in the present invention include biotin for staining with labeled streptavidin conjugate, magnetic beads (e.g., Dynabeads^{™}), fluorescent dyes (e.g., cyanine dyes, such as Cy7, Cy5, Cy3, Alexa fluor (AF) dyes such as AF 350, AF 405, AF 430, AF 488, AF 500, AF 514, AF532, AF 546, AF 555, AF 568, AF 594, AF 610, AF 633, AF 647, AF 660, AF 680, AF 700, AF 750, fluorescein, texas red, rhodamine, green fluorescent protein, and the like), radiolabels (e.g., ³H, ¹²⁵I, ³⁵S, ¹⁴C, or ³²P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads, nanocrystals, quantum dots Patents teaching the use of such labels include U.S. Patent Nos. 3,817,837; 3,850,752; 3,939,350; 3,996,345; 4,277,437; 4,275,149; and 4,366,241. A general review about labels can be found in Kricka, L. (1999), Nucleic Acid Detection Technologies - Labels, Strategies, and Formats, Clinical Chemistry 45:453-458. Further useful labels are molecule dye-quencher pairs, whereby the dye is preferentially a fluorophore. Suitable pairs are those with quenching via rapid internal energy and/or electron transfer. A preferred pair is a fluorophore-gold dye-quencher pair. In one embodiment the quencher and dye are located in the probe, whereby dye and quencher are separated by the putative mismatch position. In another embodiment quencher and dye are both located in the target and also separated by the putative mismatch position - dye and quencher have then to be introduced during an amplification step. In a further embodiment dye and quencher are on separate strands, i.e. the dye is in the probe and the quencher is in the target, and vice versa; in these embodiments the dye and quencher are also separated by the putative mismatch position. Thus it is essential in all dye-quencher pair embodiments that dye and quencher are separated by at least one nucleotide. In order to enable the rapid internal energy and/or electron transfer between dye and quencher, the maximum possible number of bases between dye and quencher varies. Upon digestion of the hybridization product with the endonuclease, quencher and dye become separated, leading to a detectable signal.
The term "signal" refers to a detectable change of physical a parameter. Appearance, disappearance, enhancement and/or reduction all represent detectable changes of the parameter.

In the figures shows
Figure 1: an exemplary array layout. All capture probes were spotted twenty times; spotting controls were spotted six times, negative and positive hybridisation probes were spotted three times. Black circles indicate the perfect match to TargetC;
Figure 2: an overview of the hybridisation experiments. Three arrays, which were hybridized with the same hybridisation solution, were treated with three different assays: 1) nuclease and enhancer, 2) nuclease, and 3) no additive;
Figure 3: the absolute fluorescence signals of the control probes after scanning the arrays with PMT35 after hybridisation and digestion (SC = Spotting control, PHC = positive hybridisation control);
Figure 4: the reduction ratios (before/after treatment) of the fluorescence signals of the control probes after digestion;
Figure 5: absolute (a) and relative (b) fluorescence signals of the capture probes. After hybridisation and digestion, the arrays were scanned with PMT50 (↓ Perfect Match probe signal, →trend of MM signal with second treatment (± Nuclease));
Figure 6: reduction ratios (before/after treatment) of absolute signal intensities of the capture probes. (↓ Perfect Match probe signal);

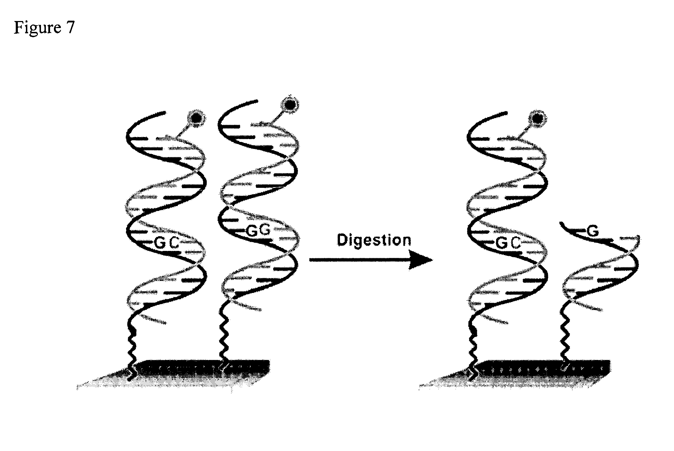

Figure 7: digestion of different mismatch combinations for the determination of a cutting matrix.

In order to perform the present method a biological sample may be used as such, i.e. without any treatment, or after isolating a nucleotide sequence contained in the sample. DNA contained in the biological sample may liberated from the cells or isolated according to techniques well known in the art, e.g. via QIAprep^{®} Spin Miniprep Kit protocol (Qiagen, Hilden, Germany). Alternative appropriate methods for obtaining DNA may be chosen, depending on the specific starting material on the basis of the knowledge of a skilled person in the art and for example disclosed in Maniatis, A Laboratory Handbook, Cold Spring Harbor, 1992. Equally, RNA may be isolated using techniques well known in the art, either utilizing commercially available kits or applying standard protocols (in Maniatis, A Laboratory Handbook, Cold Spring Harbor, 1992). Optionally, the isolated material is subjected to an amplification step. Such an isolation step assists in preventing the development of extensive background signals during the hybridization step, in case no other selection step is applied. The DNA and/or RNA contained in the sample may be also denominated as "target", "target nucleic acid" or "target molecule".

The DNA contained in the biological sample or isolated therefrom may be also amplified via a polymerase chain reaction (PCR) using one or more primers, which provides the advantage of augmenting the specific material to be investigated and to incorporate a selection step. In case of using one primer only, the complementary strand of the DNA of interest will be synthesized. Alternatively, at least two primers are utilized, allowing an exponential amplification of the material to be investigated. After the completion of the PCR reaction, the PCR product may be purified if desired. Equally, the RNA contained in the sample may be subjected to a reverse transcription reaction prior to PCR amplification.

The biological sample or the target isolated therefrom is contacted with a carrier, having on a predetermined location thereon at least one capture probe attached to the carrier by means of a spacer molecule of a defined length, under conditions permitting hybridization of complementary strands.

In the context of the invention, the nucleotides used as probes have a length from up 15 to 500 nucleotides, from up to 50 nucleotides, preferably up to 40 nucleotides, more preferably 30 nucleotides and most preferably between 15 and 30 nucleotides, such as 15, 16, 17, 18, 19, 20, 21, 22, 23, 24, 25, 26, 27, 28, 29 or 30 nucleotides. In the context of the present invention, target nucleotides are all nucleotides of any length; that is, up to the genomic sequence of an individual.

The carriers used according to the invention can be any commercially available carrier conventionally used for the purpose of hybridization, including membranes, metal carriers, plastic materials, beads or glass. A carrier with a larger number of applied nucleotides will be generally referred to as a biochip/micro-array.

The capture probes are attached to the surface of the support by means of a spacer, which does not react/interact significantly with the probe (except for fixating the probe to the carrier), target or any other DNA or RNA sequence contained in the sample. Spacer to be used in the present invention may be obtained following methods described e.g. in WO0177372, which document is incorporated herein by way of reference. The spacer molecule may be either an oligonucleotide or a polypeptide. The advantage of the first resides in the possibility to build up the spacer molecule and the probe directly on the surface of the solid support, whereas the advantage of using of a polypeptide as spacer resides in a negligible interaction with DNA.

Any process known from the prior art can be used for applying spacer molecules to the carrier, which effects temporary or permanent immobilization, fixation or adhesion of the probe nucleotide to a site or in a region of the carrier; for example, by the formation of covalent, ionic, metal-organic bonds, binding based on van der Waal's forces, or enzyme substrate interactions or so called affinity binding. Naturally, any number of spacer molecules, for example polymer-based spacers, can be arranged between the carrier and the nucleotide applied on the carrier. Furthermore, carriers based on self-assembling layer systems are also suitable for the implementation of the present invention. Application can also be done by using automatic methods.

In case of using an oligonucleotide as spacer molecule, the overall length of the capture probes (including the spacer) may range from about 30 to about 550 nucleotides, preferably of from about 30 to about 400, or 15 to 200 nucleotides, or more preferred of from about 30 to about 100, or from 30 to 50, or from 30 to 40, particularly 31, 32, 33, 34, 35, 36, 37, 38, 39 nucleotides and are fixed on a surface of any carrier. Such nucleotide sequences may be chemically synthesised oligonucleotides sequences, which may e.g. be easily performed on programmed automatic DNA synthesizer. Such sequences can bear a functionalized group (e.g. an amino groups or a hydroxyl group ) for covalent attachment upon the support, at high concentrations. Longer capture nucleotide sequences are preferably synthesised by PCR amplification (of a sequence incorporated into a plasmid containing the specific part of the capture nucleotide sequence and the non specific part (spacer)), and may subsequently be modified with a functional group to allow attachment to the surface of the carrier..

If the spacer molecule is a polypeptide, the length of the spacer molecule (without capture probe) is in range from about 5 to about 100 amino acids, preferably of from about 50 to about 50, or 5 to 30 amino acids or more preferred of from about 5 to about 20, or from 5 to 15, particularly 6, 7, 8, 9, 10, 11, 12, 13 or 14 amino acids and are fixed on a solid support. Polypeptides may be synthesized according to the state of the art. A particular suitable polypeptide is for example a thymidine spacer of the above indicated length. If the spacer molecule is an oligonucleotide, the length of the spacer molecule (without capture probe) is in range from about 3 to 50 nucleotides, 5 to 30, or 7 to 15, particularly from 9, 10, 11, 12, 13, 14 or 15 nucleotides.

The present inventors have found that the length of the above mentioned spacer has an important influence on performance of the present method, in that a too short spacer molecule does not permit the capture probe and target molecule hybridized thereon to access the active site of the endonucleases, whereas a too long spacer - in case the spacer itself is an oligonucleotide- molecule has been found to favourite digestion of single stranded DNA to a certain extent, i.e. the capture probes comprising the spacer themselves.

It will be appreciated that the length of a spacer molecule is also dependent on the length of the capture probe, specifically the position of the mismatch to be detected. Suitable lengths of a spacer molecule may be easily tested by subjecting capture probes attached to spacer molecules of different length, which in turn are attached to the surface of a solid support, to a incubation reaction with an endonucleases to be tested under appropriate conditions and monitoring if cleavage of the capture probes occurs. Alternatively, the length of the spacer molecule may be determined in silico, i.e. modelling the three dimensional structure of the endonuclease and determining therefrom the distance between the active site of the enzyme and the surface corresponding to the minimal required length. Such in silico approaches are within the knowledge of the skilled person.

The target molecule is now contacted with the surface of the support under conditions permitting hybridization of complementary strands. It will be appreciated that hybridization is performed in not a manner permitting matching of identical capture probe/ target molecule sequences only, but rather allows hybridization of DNA strains carrying a nucleotide mismatch. Suitable hybridization conditions are within the knowledge of the skilled person and may be derived e.g. from Maniatis, A Laboratory Handbook, Cold Spring Harbor, 1992.

The results of the previous step may be optionally analyzed by determining whether hybridization occurs and at which position on the array, i.e. by subjecting the array to a conventional detection and computerized evaluation. The advantage of such a previous analyzing step of the array resides in that the subsequent step of the endonucleases treatment and the final analyzing represent a "control step". With other words, the present invention may be used as a simple and inexpensive method to control the results obtained from a method according to the state of the art suitable for determining point mutations. A further advantage resides in that the present method may be also used for determining optimal hybridization conditions for arrays suitable to detect point mutations in that conditions are obtained permitting a method in which the hybridization conditions favorite exactly matching between complementary strands and/or matching with a single mismatch only.

The carrier is than contacted with a nuclease under conditions permitting the nuclease to digest hybridized strands in at least two DNA fragments. A suitable endoculease may be a mismatch specific plant DNA endonuclease belonging to the CEL family of plant endonucleases that cleave DNA at sites of base-substitutions and other distortions (Oleykowski, C.A. et al., Nucleic Acids Research, 26 (1998) 4597-4602 and Yang, B. et al., Biochemistry, 39 (2000) 3533-3541). Such an enzyme is commercially available as Surveyor Nuclease capable to cut both strands of a DNA heteroduplex are on the 3'-side (Qiu, P. et al., Biotechniques, 36 (2004) 702 et sqq. and Sokurenko, EV. et al., Nucleic Acids Research, 29 (2001) 111). Insertion/deletion mismatches as well as all base-substitutions are recognized.

Other suitable endonucleases comprise S1 Nuclease (Promega GmbH, Mannheim, Germany), which also exhibits the capability to digest mismatch pairings (Gaylord, B.S. et al., Proceedings of the National Academy of Sciences of the United States of America, 102 (2005) 34-39 and Till, B.J. et al., Nucleic Acids Res., 32 (2004) 2632-2641). Other suitable enzymes are P1 nucleases and mung bean nuclease. Suitable conditions are known to the skilled person and may be easily tested.

The nuclease's activity may be amplified by means of a nuclease enhancer obtainable from e.g. Transgenomic, Alancourt, France.

In case Surveyor endonuclease is employed the spacer may be a thymidine spacer comprising 9 to 13 thymidine residues, preferably, 10 to 12 thymidine residues and more preferably 11 thymidine residues in length to which the capture probes are attached.

After incubation of the carrier with endonuclease occurrence and/or location of said at least two DNA fragments is determined by conventional means known to the person having skills in micro-array technology. Said occurrence and/or location of said at least two DNA fragments is indicative of the presence of a point mutation in that e.g. disappearance of a signal previously present at a particular position may indicate that the endonuclease has cleaved two complementary strands carrying a single mismatch. The position(s) at which a signal disappearance has occurred is indicative for the presence of a particular point mutation.

According to an embodiment of present invention the spacer molecule comprises between 5 to 30 nucleotides or 5 to 15 amino acids.

According to another embodiment of present invention the carrier is a biochip or a micro-array.

According to an embodiment the nucleotide sequence applied to the carrier is a DNA..

The principle the method according to the present invention may be used in any detection method known to date in the art, which is based on the method of hybridization of complementary nucleotide strands. According to an embodiment, the present method is used for determining genetic mutations in organisms. Examples of such mutations include point mutations giving e.g. raise to antibiotic resistance in bacterial strains. Furthermore, the method according to the invention is also suitable for the determination of genetic mutations, which occur in any number of organisms, including mammals and human beings. Similarly, for example, even synthetically manufactured nucleotide sequences can be investigated using the method according to the invention.

The method according to the invention is particularly used in the examination of a nucleotide for the presence of a single nucleotide polymorphism or SNP. SNP is defined as any polymorphism between two genomes, which is based on a single nucleotide substitution, a minor deletion or insertion. Specific SNPs are known to be correlated with an increased risk for the occurrence of a particular disease and are therefore of diagnostic interest.

According to still another embodiment, the present method is used for determining, resistance to antibiotics. Examples of resistances to antibiotics comprise β-lactam antibiotic resistance, penicillin antibiotic resistance, cephalosporin antibiotic resistance, fluoroquinolone resistance or ampicillin resistance.

According to another embodiment the capture probe is labeled. Examples for such detectable labels comprise biotin for staining with labeled streptavidin conjugate, magnetic beads, fluorescent dyes, radiolabels, enzymes and colorimetric labels. Preferably, the capture probe is radioactive-labeled or fluorescence-labeled. Labeling of DNA may be performed e.g. during an amplification step by including in the amplification process nucleotides harboring an appropriate label. Alternatively, a label may be attached to the nucleic acid, including, for example, nick translation or end-labeling by attachment of a nucleic acid linker joining the sample nucleic acid to a label.

According to a preferred embodiment of the present invention a Kit for implementing the present method is provided. The kit comprises as constituents an array, which comprises capture probes arranged at pre-determined locations, said capture probes being covalently bound to a carrier via a spacer molecule of a defined length; a nuclease; and optionally, buffers, labels, glycoylase and a manual. The spacer molecule preferably comprises between 5 to 30 nucleotides or 5 to 15 amino acids. The glycosylase, an enzyme which removes the base at a mismatch and facilitates the later action of an endonuclease, may be included in the reaction in order to enhance the reaction.

According to an embodiment, the kit is suitable for determining genetic mutations in organisms in that the kit may comprise a set of capture probes forming subsets of four capture probes, in which subset a particular nucleotide is present in all four possible variants whereas the remaining sequence of the capture probes in the subset is left identical. The kit is preferably used for determining resistance to antibiotics. Preferred examples of resistances to antibiotics comprise β-lactam antibiotic resistance, penicillin antibiotic resistance, cephalosporin antibiotic resistance, fluoroquinolone resistance or ampicillin resistance.

According to another preferred embodiment the capture probes comprised in the kit are labeled with a detectable label. The capture probes are preferably radioactive-labeled or fluorescence-labeled.

The following examples explain the invention, while not limiting it. In particular, the sequence lengths described therein of the first and second nucleotide and the dye labeling of the second nucleotide do not limit the present invention.

### Examples

### Array fabrication

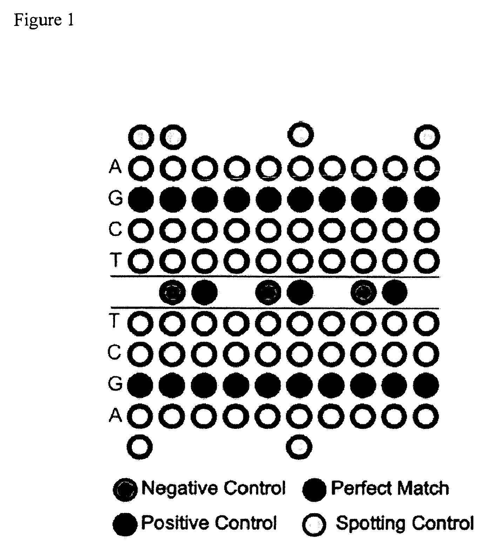

All probes (Table 1) were purchased from Metabion (Martinsried, Germany) in HPLC purity grade were dissolved in spotting buffer to a final concentration of 20 pM and spotted in a MicroGrid II (BioRobotics, Cambridge, United Kingdom) on epoxycoated slides. The array layout is shown in Figure 1. After spotting, the slides were incubated at 30 min at 60 °C in a drying compartment (Memmert, Schwabach, Germany) for covalent coupling of the amino-modified probes to the expoxy-coated surface. Before hybridisation, the slides were rinsed in 0.1 % (vlv) of 10 Triton X-100 for 5 mm, in 0.5 ml concentrated HCI per 1 of ddH₂O for 4 mm, in 100 mM KCI for 10 min and finally in ddH₂O Subsequently, the arrays were blocked for 15 min at 50 °C in blocking solution (25 % *(vlv)* ethylene glycol, 0.5 ml concentrated HCl per 1 of ddH₂O in a heating compartment (OV5; Biometra, Göttingen, Germany).

Finally, the slides were rinsed for 1 min. in ddH₂O and dried with nitrogen flow.

**Table 1 Capture probe and target sequences**

| | | |
|---|---|---|
| Oligonucleotide name | 5'-3' sequences ^{a} | |
| Spotting control | Cy3-TCTAGACAGCCACTCATA | |
| Positive hybridisation control | GATTGGACGAGTCAGGAGC | |
| Negative hybridisation control | TCTAGACAGCCACTCATA | |
| TargetC | Cy3-CGAGCAGAAQCATCGCAGC | |
| FimH_551A | GCTGCGATGATTCTGCTCG | MM |
| FimH_551G | GCTGCGATGGTTCTGCTCG | PM |
| FimH_551C | GCTGCGATGCTTCTGCTCG | MM |
| FimH_551T | GCTGCGATGJTTCTGCTCG | MM |

| | | |
|---|---|---|
| ^{a} Capture probes were modified at the 5'-end with a 11-thymidine spacer and a C₆-amino modification. | | |

### Hybridisation and washing

For hybridisation, 0.05 pmol of TargetC was spiked with 0.05 pmol positive hybridisation control DNA, diluted in 25 µl of 6x SSPE (1x SSPE is 0.18 M NaCI, 10 mM NaH₂PO₄, and 1 mM EDTA [pH 7.0]), hybridized within a gene frame (0.9 x 0.9 cm; ABgene House; Hamburg; Germany) and closed by a coverslip (ABgene House). Hybridisation was performed by incubating in a thermomixer with an exchangeable thermoblock for slides (Eppendorf) for two hours at 50 °C. After hybridisation , the slides were successively washed with 2x SSC (0.3 M NaCl and 0.03 M sodium citrate) / 0.1 % sodium dodecyl sulfate, 2x SSC and finally with 0.2x SSC each for 10 min before being dried with nitrogen flow. The array was scanned with ScanArray Express (below) before being treated with the nuclease.

### Nuclease activity on chip

After washing, the digestion mixture containing 1 µl Surveyor Nuclease, 1 µl Nuclease Enhancer (Transgenomic, Alancourt, France) and 1x Taq buffer (Eppendorf, Hamburg, Germany) was applied on the array within a gene frame and closed by a coverslip. In a second assay, the Enhancer was left out. As control, a mixture containing only 1x Taq buffer was applied on a separate array. The digestion was done at 42 °C for 20 min by incubating in a thermomixer with an exchangeable thermoblock for slides (Eppendorf). After digestion, the slides were again washed with 0.2 x SSC for 10 min before being dried with nitrogen flow and scanned with ScanArray Express

### Image acquisition and data analysis

The fluorescence signals of the arrays after hybridisation (SH) and digestion (SD) were acquired with ScanArray Express (Perkin Elmer) using 90 % laser power and PMT 50, respectively PMT35 for the control probes. The quantification of fluorescence signal intensities was performed with ScanArray Express software (Perkin EImer). For further analysis, the mean signal intensity minus local background intensity was processed in Excel software and the average value of each capture probe was calculated. The maximum average signal was termed as perfect match (PM), whereas the other three oligonucleotide probes with a lower signal were referred to as mismatches (MM5). For normalization, the relative intensity (RI) was calculated by dividing the average net signal of each probe by the signal of the average of the respective PM. The signal reduction (SR) by the influence of the nuclease was calculated by SR = 1 -SH /SD for every spot and the average signal reduction was determined subsequently.

### Influence of nuclease treatment on control signals

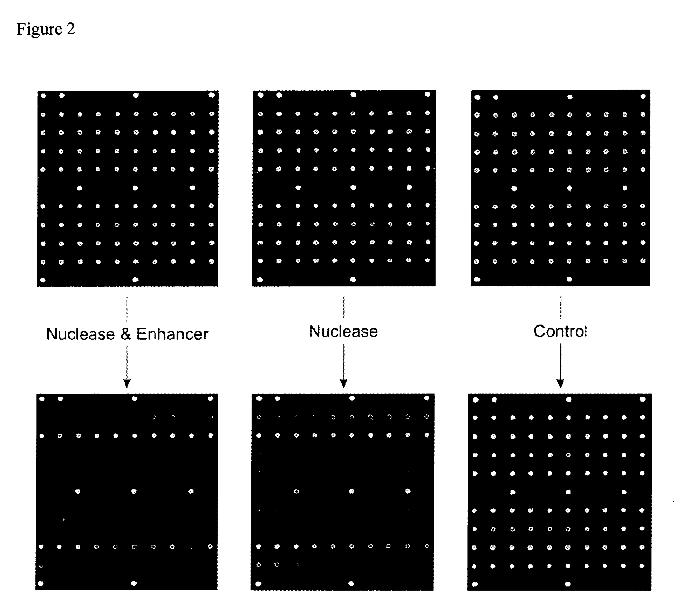

After hybridisation, three separate arrays were treated with a different digestion assay. The first array was incubated with a solution containing the nuclease as well as the enhancer, the second array was treated just with the nuclease and the third one was used as a negative control (Figure 2). After hybridisation and digestion, the arrays were scanned using PMT35 in order to avoid signals in the saturated range for the control probes.

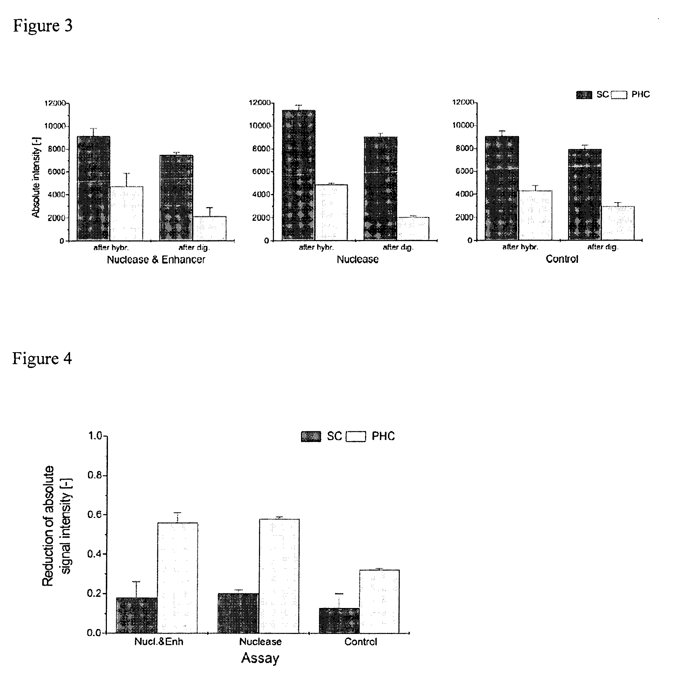

The absolute signal intensities are shown in Figure 3 whereas the reduction of the respective signals is summarized in Figure 4. It is noteworthy that all signals are reduced by the additional treatment independent from the solution composition which can be shown by the low, but significant reduction observed for the probes of the control experiments.

Two additional conclusions can be made: 1.) There is an unambiguous difference in the reduction of the signal between spotting control (labelled single-stranded DNA) and positive hybridisation control (DNA/DNA hybrid of unlabeled probe and labelled complementary DNA). This might be due to the fact that the additional incubation and washing time result in a higher stringency that causes lower signals. Further investigations will be conducted to elucidate this further. 2.) The reduction ratio observed for the positive hybridisation control is significantly lower (0.32) than for the treated probes (0.57). These results imply that the nuclease does not only cut mismatch hybrids but also perfect match hybrids and that in our approach, no single strand cutting activity could be observed. The enhancer did not seem to have any effect on the digestion on surface.

### Influence of nuclease on capture probe signals

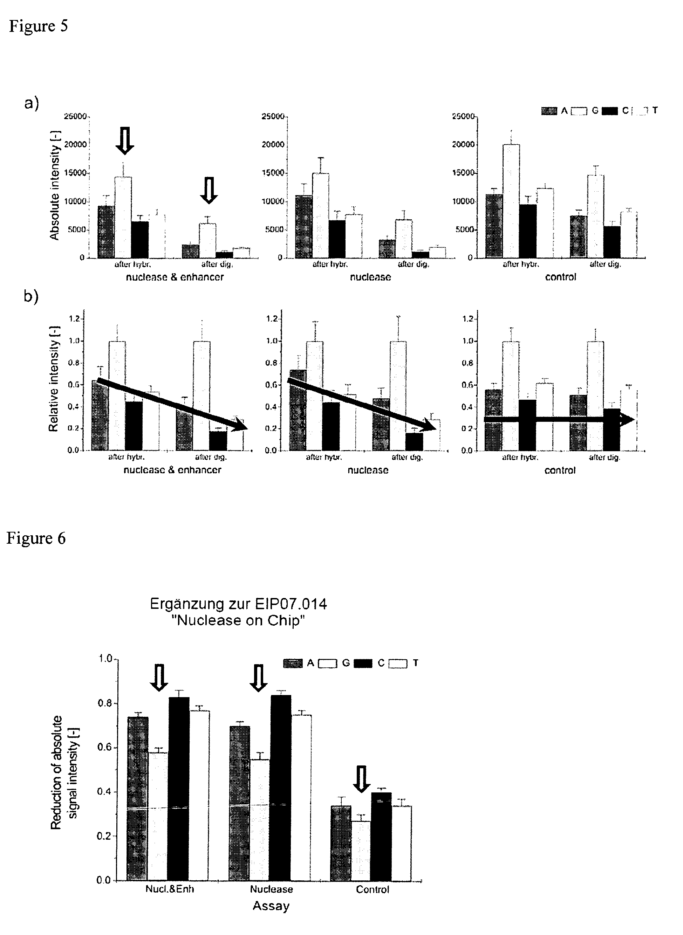

The influence of the nuclease treatment on the discrimination between perfect matched DNA/DNA hybrids and single nucleotide mismatched DNA/DNA hybrids may be seen in Figure 5. In accordance to the results for the control probes, all probes showed lower absolute signals after additional incubations (Figure 5 a). The big difference was found when the relative signals (RI, Figure 5 b) were considered (as performed in SNP detection arrays). No difference for the RIs where found in the control experiment without nuclease treatment. In contrast, the incubation with nuclease considerably lowered the mismatch signals as compared to the perfect match ones.

The data shown in Figure 6 suggest a cutting preference of the nuclease for C-C mismatches (0.83) over T/A-C mismatches (0.76, 0.72 respectively). The perfect match signals were also reduced (0.56) which indicates a non optimal reaction condition. However, the preference of mismatch hybrids for the activity of the nuclease is unambiguous. According to the data obtained by the control probes, the enhancer did not seem to have any positive or negative effect on the activity and specificity of the nuclease.

## Claims

1. A method for determining a point mutation, said method comprising the steps of:
(i) obtaining a biological sample;
(ii) optionally isolating a nucleotide sequence contained in the sample;
(iii) contacting the biological sample or the nucleotide sequence isolated from the sample with a carrier, having on a predetermined location thereon at least one capture probe attached to the carrier by means of a spacer molecule of a defined length, under conditions permitting hybridization of complementary strands;
(iv) optionally determining whether hybridization occurs and at which position on the array;
(v) contacting the carrier with an endonuclease under conditions permitting the endonuclease to digest hybridized strands in at least two DNA fragments;
(vi) determining occurrence and/or location of said at least two DNA fragments, wherein the occurrence and/or location of said at least two DNA fragments is indicative of the presence of a point mutation.

2. The method according to claim 1, wherein the spacer molecule comprises between 5 to 30 nucleotides or 5 to 15 amino acids.

3. The method according to claim 1 or 2,-wherein the carrier is a biochip or a micro-array.

4. The method according to one of the preceding claims, wherein the nucleotide sequence brought in contact with the carrier is a DNA.

5. The method according to one of the preceding claims for determining genetic mutations in organisms.

6. The method according to one of the preceding claims for determining resistance to an antimicrobial agent or an antitumour agent.

7. The method according to claim 6, wherein the antimicrobial agent is selected from the group of antibiotics, antifungals, antivirals and antiparasitic drugs.

8. The method according to Claim 7, wherein the resistance to antibiotics is β-lactam antibiotic resistance, penicillin antibiotic resistance, cephalosporin antibiotic resistance, fluoroquinolones resistance or ampicillin resistance.

9. The method according to one of the preceding claims, wherein the capture probe is labeled.

10. The method according to one of claims 1 to 8, wherein the nucleotide sequence is labeled.

11. The method according to one of claims 9 or 10, wherein a radioactive or a fluorescence label is used.

12. The method according to one of claims 1 or 11, wherein the endonuclease remains active within a temperature range of 40 °C to 95 °C, preferably between 40 °C to 70 °C and most preferably between 40 °C to 60

13. A Kit for implementing a method according to one of the preceding claims, comprising:
an array comprising capture probes arranged at pre-determined locations, said capture probes being covalently bound to a solid support via a spacer molecule of a defined length;
an endonuclease; and
optionally, buffers and labels.

14. The kit according to claim 13 comprising a glycosylase

15. The kit according to claim 13, wherein the spacer comprises between 5 to 30 nucleotides or 5 to 15 amino acids.

16. The kit according to claim 13 or 15 for determining genetic mutations in organisms.

17. The kit according to claims 13 to 15 for determining resistance to an antimicrobial agent or anti-tumour agent.

18. The kit according to Claim 17, wherein the resistance to antibiotics is β-lactam antibiotic resistance, penicillin antibiotic resistance, cephalosporin antibiotic resistance, fluoroquinolones resistance or ampicillin resistance.

19. The kit according to claims 13 to 18, wherein the capture probe is labeled.

20. The kit according to claim 19, wherein the capture probe is radioactive-labeled or fluorescence-labeled.
